(19) Europäisches Patentamt / European Patent Office / Office européen des brevets

(11) EP 2 793 577 B1

(12) # EUROPEAN PATENT SPECIFICATION

(45) Date of publication and mention of the grant of the patent:
**12.04.2017 Bulletin 2017/15**

(21) Application number: **12798329.4**

(22) Date of filing: **11.12.2012**

(51) Int Cl.:
***A01N 31/08*** (2006.01) ***A01P 1/00*** (2006.01)

(86) International application number:
**PCT/EP2012/075109**

(87) International publication number:
**WO 2013/092311 (27.06.2013 Gazette 2013/26)**

(54) **ANTIMICROBIAL METHOD AND COMPOSITION**

ANTIMIKROBIELLES VERFAHREN UND ZUSAMMENSETZUNG

PROCÉDÉ ANTIMICROBIEN ET COMPOSITION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2011 EP 11195245**

(43) Date of publication of application:
**29.10.2014 Bulletin 2014/44**

(73) Proprietors:
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**
- **Unilever PLC**
  **London, Greater London EC4Y 0DY (GB)**
  Designated Contracting States:
  **CY GB IE MT**

(72) Inventors:
- **CORNMELL, Robert, Joseph**
  **Bebington**
  **Wirral**
  **Merseyside CH63 3JW (GB)**
- **GOLDING, Stephen**
  **Bebington**
  **Wirral**
  **Merseyside CH63 3JW (GB)**
- **STOTT, Ian, Peter**
  **Bebington**
  **Wirral**
  **Merseyside CH63 3JW (GB)**
- **THOMPSON, Katherine, Mary**
  **Bebington**
  **Wirral**
  **Merseyside CH63 3JW (GB)**

(74) Representative: **van den Brom, Coenraad Richard**
**Unilever Patent Group**
**Olivier van Noortlaan 120**
**3133 AT Vlaardingen (NL)**

(56) References cited:

| | |
|---|---|
| **WO-A1-2013/083581** | **WO-A1-2013/083587** |
| **WO-A1-2013/083588** | **WO-A2-2013/083586** |
| **DE-A1- 2 263 126** | **FR-M- 1 137** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 793 577 B1

## Description

### FIELD OF THE INVENTION

The present invention relates to a method for disinfecting a surface. The invention particularly relates to a non-therapeutic method for disinfecting a surface on the human body. The invention also relates to an antimicrobial composition.

### BACKGROUND TO THE INVENTION

Sanitising and disinfecting soap or cleaning compositions are of great benefit to individuals, since proper use generally may reduce the number of germs and pathogens the individual is exposed to. Thus, such compositions may for instance play an important role in reducing the occurrence and spread of infectious diseases. Sanitising and disinfecting soap compositions comprising chlorine-based antimicrobial agent such as triclosan are known. Such compositions require a rather long contact time to provide efficacious antimicrobial action. In practice, users, in particular children, do not spend a long time on cleansing and as a result cleaning with such compositions does not provide adequate prevention from surface or topical infection or adequate protection against diseases. The user, in spite of cleaning his hands, is generally likely to end up with relatively inadequate bacterial removal from his skin. Therefore, he may cause contamination of further animate and/or inanimate surfaces and contribute to the spreading of pathogens and consequent diseases. Users in general and children in particular who wash contaminated hands before meals with slow-acting antimicrobial compositions for a relatively short time are at risk of contracting diseases.

Similarly in the area of hard surface cleaning, e.g. cleaning of floors, table tops or utensils, the antimicrobial in the compositions are in contact with the substrate for less than a few minutes after which the surface is either wiped off or rinsed with water. These short time scales of cleaning action are ineffective in providing the desired benefit since most known antimicrobials commonly used in such products take several hours to provide the desired kill of microbes.

Therefore, there is a need of providing a method for disinfection and an antimicrobial composition usable in such a method, wherein the method provides more efficacious antimicrobial action during a relatively short cleaning period, preferably about 30 seconds or less.

A well-established class of antimicrobially active compounds are phenolic compounds. [P A Goddard and K A McCue in "Disinfection, Sterilisation and Preservation", ed. S S Block, 5th edition, Lippincott, Williams and Wilkins, Philadelphia, 2001 pp. 255-282.] However, not every phenolic compound is suitable as an antimicrobial agent. Moreover, many phenols - even if they are antimicrobially active - may exhibit undesirable side-effects, such as corrosiveness, malodour and irritating or sensitising effects when applied on the human or animal skin. In addition, small changes to molecular structure may in general strongly affect both the desired and the undesired effects in a generally unpredictable way.

A particularly well-known group of phenolic compounds are halogenated phenolics. However, there are concerns about the usage of chlorinated phenols as antimicrobials and herbicides, since they are perceived as pollutants, and after often associated with environmental toxicity, bioaccumulation and low biodegradability. [JP Voets et al, J. Appl. Bact. vol 40, p. 67-72 (1976); "Chlorophenols in the terrestrial environment" J Jensen, Reviews of environmental contamination and toxicology, Vol 146 pp 25-51 (1996)]

The patent applications FR 1137 M, FR 1.313.717 and BE 591.064 disclose the bacteriostatic properties of cyclohexyl-derivatives of phenol, such as cyclohexyl-methyl phenols.

DE2263126A1 relates to antibacterial medicaments, such as ointments, that may be applied to the skin. The antibacterial compounds disclosed in DE2263126A1 are phenols substituted at least with a cyclic $C_5$ substituent and two alkyl groups and optionally a halogen atom.

Despite the general availability of antimicrobial compounds and compositions, there remains a continuous need to find new and alternative antimicrobial compositions and active compounds that are suitable for use in such compositions. Such alternatives may reduce the dependency on current raw materials. Moreover, in the field of antimicrobials, the availability of alternatives may reduce the risk of development of microbial resistance or insensitivity to particular antimicrobial compounds.

In addition, there is a continued need to reduce the total amount of active ingredients required in such an antimicrobial composition. This need may for instance be driven by the desire for cost-efficiency, because such compositions are particularly relevant to developing countries. Moreover, reducing the amounts may also be beneficial for environmental reasons.

A particular problem of certain antimicrobial actives, and especially some phenolic compounds is that they are generally well-perceptible due to their olfactory properties. Although the latter - at least for some species - are appreciated in some fragrance compositions, they are generally considered too intense by some users when they are applied at concentrations efficacious in rapid disinfection. Additionally, a lower concentration of odoriferous compounds or the

availability of antimicrobial compounds that are less odoriferous allows greater flexibility to the manufacturer in providing alternative scents to his composition at lower doses. Hence there is a need to provide alternatives that preferably require lower concentrations and/or have a more acceptable sensory profile.

In view of the above-observed problems and drawbacks of the prior art, it is an object of the present invention to provide a non-therapeutic method for sanitising and/or disinfecting, in particular of surfaces.

It is a particular object of the present invention to provide a non-therapeutic method for disinfecting surfaces based on a new antimicrobial composition.

It is another object of the invention to provide such a method, requiring a lower dose of antimicrobial composition.

It is yet another object of the invention to provide a non-therapeutic method for disinfection with a reduced disinfection time.

Similarly, it is an object of the present invention to provide a non-therapeutic method based on an antimicrobial composition in which the olfactory contribution of the antimicrobially active compounds is reduced or in which the active compound contributes to providing a consumer-acceptable or even consumer-appreciated scent.

In particular, it is an object of the invention to provide a non-therapeutic method for disinfection that gives improved disinfection during cleansing of surfaces, in particular hard surfaces or surfaces of the human body, such as the skin and the oral cavity.

In view of the other objects, it is also an object of this invention to provide antimicrobial compositions that are applicable in a method for disinfection of a surface.

It is a further particular object of the invention to provide such antimicrobial compositions that contribute to reducing the required contact time in a method for disinfection of a surface.

## SUMMARY OF THE INVENTION

We have now found that one or more of the above objects are met by the present invention. Thus, we have now found that a method for disinfection involving the step of applying an antimicrobial composition comprising cyclopentyl- or cyclopentenyl-substituted phenols provides for similar or more efficacious anti-microbial action at similar or lower concentrations of the applied composition when compared to other phenols, such as for instance cyclohexyl phenols. Moreover, the cyclopentyl- or cyclopentenyl-substituted phenols only have a weak odour. We have consequently also found an antimicrobial composition comprising cyclopentyl- or cyclopentenyl-substituted phenols, whereby the composition is applicable in foresaid method.

Accordingly, in a first aspect the invention provides a non-therapeutic method of disinfecting a surface comprising the steps of

a. applying to the surface an antimicrobial composition comprising

    i. 0.001 to 5% by weight of one or more substituted phenols, and
    ii. a carrier; and

b. removing the composition from the surface;

wherein the one or more substituted phenols have the following structure

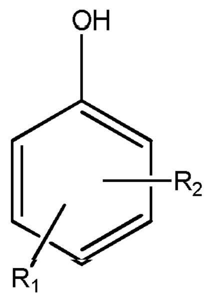

wherein

$R_1$ is a cyclopentyl group or a cyclopentenyl group, and wherein
$R_2$ is independently selected from the group consisting of

hydrogen,
linear or branched $C_1$ to $C_5$ alkyl, and
linear or branched $C_2$ to $C_5$ alkenyl.

According to a second aspect of the invention, there is provided an antimicrobial composition comprising

a. 0.001 to 5% by weight of one or more substituted phenols,
b. a carrier, and
c. from 1 to 80% by weight of one or more surfactants;

and wherein the one or more substituted phenols have the following structure

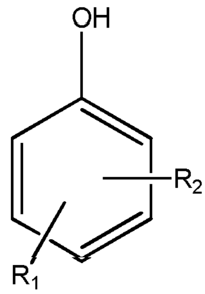

wherein

$R_1$ is a cyclopentyl group or a cyclopentenyl group, and wherein
$R_2$ is independently selected from the group consisting of

hydrogen,
linear or branched $C_1$ to $C_5$ alkyl, and
linear or branched $C_2$ to $C_5$ alkenyl.

## DETAILED DESCRIPTION

For the avoidance of doubt, any feature of one aspect of the present invention may be utilised in any other aspect of the invention. The word "comprising" is intended to mean "including" but not necessarily "consisting of" or "composed of." Thus, the term "comprising" is meant not to be limiting to any subsequently stated elements but rather to optionally also encompass non-specified elements of major or minor functional importance. In other words, the listed steps or options need not be exhaustive. Whenever the words "including" or "having" are used, these terms are meant to be equivalent to "comprising" as defined above. It is noted that the examples given in the description below are intended to clarify the invention and are not intended to limit the invention to those examples per se.

Unless specified otherwise, numerical ranges expressed in the format "from x to y" are understood to include x and y. When for a specific feature multiple preferred ranges are described in the format "from x to y", it is understood that all ranges combining the different endpoints are also contemplated.

Phenol in its strict sense refers to hydroxybenzene ($C_6H_5OH$), but where appropriate the word phenol may in its wider meaning as understood by the skilled person also refer to a member of the class of compounds which comprise at least one such a characteristic hydroxybenzene moiety.

Throughout this description, the term disinfection refers to a reduction of the number of viable microorganisms in a given medium or on a given surface by physical or chemical means. Typically, disinfection involves the destruction or inactivation of said microorganisms.

The antimicrobial method involves a composition comprising one or more substituted phenols and a carrier. Various steps of the method and various components of the antimicrobial composition are described below. The compositions of the present invention are preferred for non-therapeutic use. The compositions and the non-therapeutic method of the present invention are more particularly preferred for use in cleaning surfaces of human body including skin, hair or oral cavity or for hard surface cleaning applications.

*Method of disinfecting a surface*

According to the first aspect of the invention, there is provided a method of disinfecting a surface. Preferably, the surface is skin. Thus, for example, a surface like the hands, face, body, or the oral cavity is contacted with the composition of the invention. Typically, such hard surfaces are surfaces that commonly require cleaning and preferably also require sanitisation or disinfection. Such surfaces may be found in many household or industrial environments, and may include for example kitchen and bathroom surfaces, table tops, floors, walls, windows, utensils, cutlery, and crockery. Such surfaces may be made from many different materials, such as for instance plastics, wood, metal, ceramics, glass, concrete, marble, and painted surfaces.
The method according to the first aspect of the present invention includes the step of applying to the surface an antimicrobial composition comprising

a. 0.001 to 5% by weight of one or more substituted phenols, and
b. a carrier.

The composition may be applied to the surface by any suitable means known to the skilled person. For instance, a suitable means may be pouring, dropping, spraying or wiping in case of liquid compositions.
Preferably, the method includes diluting or dissolving the composition with a suitable solvent, preferably water, before or whilst applying the composition to the surface. Such dissolving is preferred in particular in case the composition is a solid composition. Alternatively, solid compositions may also be directly spread, rubbed, or sprayed, e.g. in the form of a powder.

*Substituted phenols of step (i)*

The one or more substituted phenols of the composition to be applied are described here, the carrier is described below. The antimicrobial composition to be applied comprises 0.001 to 5% by weight of one or more of the substituted phenols. The composition comprises preferably 0.005 to 4.5 wt-%, more preferably 0.01 to 4 wt-%, even more preferably 0.02 to 3 wt-%, yet more preferably 0.03 to 2 wt-%, still more preferably 0.04 to 1 wt-%, even more preferably 0.05 to 0.75 wt-% and still more preferably 0.1 to 0.5 wt-% of the substituted phenols. The concentration of the substituted phenols in the antimicrobial composition is preferably such that, when the composition is diluted or dissolved with a suitable medium during use, (e.g. when washing hands with water and a composition according to the invention) the concentration in the diluted or dissolved mixture is still sufficient to be antimicrobially efficacious.
The substituted phenol may be a single compound or may be a mixture of substituted phenols as detailed below. In case the composition according to the invention comprises a mixture of such substituted phenols, the mixture preferably comprises at least 30%, more preferably at least 50%, even more preferably at least 70% and still more preferably at least 90% by weight of one substituted phenol with respect to the total weight of the substituted phenols.
At concentration ranges of the one or more substituted phenols below their lower concentration limits, the desired fast acting antimicrobial kinetics would not be met. At concentrations higher than the higher preferred concentrations of the substituted phenols, while the kinetics of action would not be compromised, the present inventors have found that unlike in therapeutic/pesticidal/herbicidal applications where sensorial aspects are not critical, in the present application, which is preferably a personal cleaning, oral care or hard surface cleaning application, the product is in contact with hands, mouth or other body parts, the sensorial aspects like smell and skin feel would be compromised. Thus, the composition should preferably not be sensorially unpleasant.
The one or more substituted phenols have the following structure

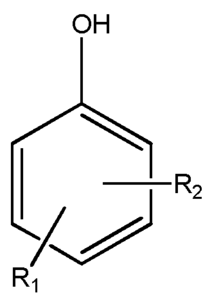

wherein $R_1$ is a cyclopentyl group or a cyclopentenyl group.

Preferably, $R_1$ is a cyclopentyl group.

The cyclopentyl group or the cyclopentenyl group may be substituted to any of the carbons of the phenol ring with positions 2, 3, 4, 5, or 6 with respect to the hydroxyl group. The cyclopentenyl group comprises a C-C double bond, which may take any position in the ring. Thus, it may be a (cyclopent-1-en-1-yl)-, a (cyclopent-2-en-1-yl)-, (cyclopent-3-en-1-yl)- group.

Therefore, the substituted phenols according to the present invention are for instance selected from compounds having one of the structures in Table 1.

**Table 1**

| cyclopentyl-substituted phenols | cyclopentenyl-substituted phenols |
|---|---|
| 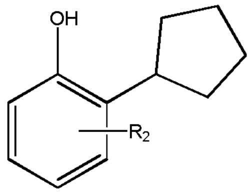 | 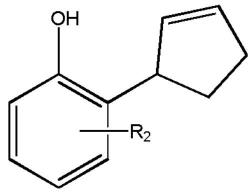 |
| 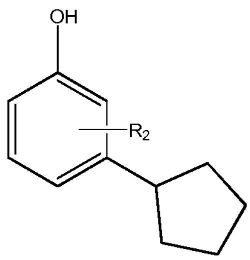 | 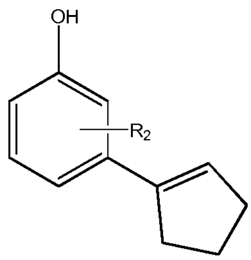 |
| 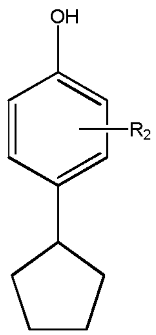 | 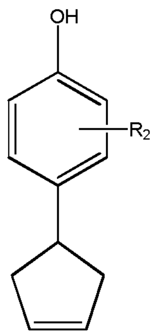 |

The substituent $R_2$ is independently selected from the group consisting of hydrogen, linear or branched $C_1$ to $C_5$ alkyl, and linear or branched $C_2$ to $C_5$ alkenyl.
Thus, $R_2$ may for instance be a methyl, ethyl, propyl, isopropyl, *tert*-butyl, allyl, or (pent-3-en-2-yl) group. The $R_2$ group may be substituted to any of the carbons of the phenol ring not bearing the OH or the ring substituent.

In case the carrier or the dissolution medium during later application is water-based, it may be advantageous if the one or more substituted phenols are sufficiently water-soluble. The substituted phenols are sufficiently water-soluble if they are soluble to at least the minimum concentration required in the antimicrobial composition according to the invention. Therefore, preferably, $R_2$ is selected from the group consisting of hydrogen, linear or branched $C_1$ to $C_3$ alkyl, and linear or branched $C_2$ to $C_3$ alkenyl. More preferably, $R_2$ is hydrogen or methyl. Even more preferably, $R_2$ is hydrogen.

Combinations of preferred $R_1$ and preferred $R_2$ substituents are particularly preferred. Therefore, it is particularly preferred that the one or more substituted phenols are selected from the group consisting of 2-cyclopentylphenol, 4-cyclopentyl phenol, and mixtures thereof. The latter compounds have the structures shown in Table 2. These compounds may be used individually, but mixtures may advantageously be sourced, since typical synthetic routes yield a mixture of 2-cyclopentylphenol and 4-cyclopentyl phenol.

**Table 2**

| 2-cyclopentylphenol | 4-cyclopentyl phenol |
|---|---|
| 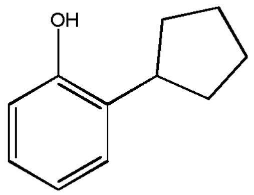 | 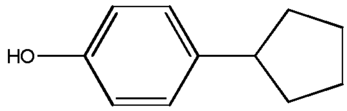 |

Mixtures of preferred substituted phenols are also preferred. Where applicable, the different stereoisomers of the substituted phenols are contemplated. Thus, compositions comprising enantiomerically pure radicals, racemic mixtures and other mixtures of different stereoisomers are equally preferable.

Without wishing to be bound by theory, it is believed that the mode of antimicrobial action of the substituted phenols according to the present invention is similar.

Suitable substituted phenols according to the present invention may be commercially sourced or obtained via synthetic chemical methods. Such methods are generally well-known to the person skilled in the art. For example, the synthesis of cyclopentyl-phenols is disclosed by Phukan and Sudalai [P. Phukan and A. Sudalai; J. Chem. Soc., Perkin Trans. 1, (1999), p.3015] and the synthesis of cyclopentenyl-phenols is disclosed by Dai *et a.* [S. H. Dai, C. Y. Lin, D. V. Rao, F. A. Stuber, P. S. Carleton, and H. Ulrich; J. Org. Chem. vol 50, p.1722 (1985)].

*Method of disinfecting a surface - rinsing step*

The method according to the first aspect of the present invention also includes the step of removing the composition from the surface. Here, removing the composition also encompasses partially removing the composition. Preferably, the step of removing the composition comprises rinsing the surface with a suitable solvent or wiping the surface with a suitable wipe, more preferably, this step consists of rinsing the surface with a suitable solvent or wiping the surface with a suitable wipe.

The solvent for rinsing the surface is preferably water but could also be for example a mixture of water and alcohol. It is then rinsed preferably with sufficient amounts of water after a pre-determined period of time to remove any visible or sensory residue of the composition. Alternately, an alcohol wipe or a water/alcohol impregnated wipe may be used to wipe the surface to be visibly free of the anti-microbial composition. The step of rinsing the substrate is preferably carried out less than 5 minutes, more preferably less than 2 minutes, further more preferably less than a minute and in many cases even more preferably less than 15 seconds after the step of applying the composition on the substrate.

*Disinfection time*

These times between applying the composition and rinsing or wiping are preferably related to the disinfection time, in order to ensure optimal cleansing and sanitising of the surface. Therefore, the invention preferably relates to a method, wherein the disinfection time T of said method is less than 300 seconds, preferably less than 60 seconds, and more preferably less than 15 seconds; wherein T is defined as the time that elapses from the moment of adding the composition to a microbial culture until the number of microbes per unit volume of the culture is reduced by a factor of 100 000; and wherein the initial number of microbes preferably exceeds about 100 000 000 microbes per millilitre and wherein the composition is preferably a liquid composition.

The disinfecting action (as may be expressed in terms of the disinfection time T) of the method is preferably determined according to the protocol of Example 1 as described hereinafter. This test relates to a standardised test environment in which the microbial culture is kept in suspension. A similarly suitable test is the standard suspension method described in European Standard EN1276, with the proviso that the disinfection time is adapted to suit the above criteria as will be clear to a person skilled in the art. Alternatively, one of the test methods as described in WO 2010/046238 may for instance be applied to establish the disinfecting action. Such test methods may preferably also be used by the skilled person to determine the optimal concentrations of the one or more substituted phenols in an antimicrobial composition according to the present invention.

Alternatively, since the method is directed towards surface disinfection, the disinfection time may also be determined by test methods involving a surface. Therefore, the invention preferably relates to a method according to the present invention, wherein the surface disinfection time T2 of said method is less than 60 seconds, preferably less than 15 seconds, wherein T2 is defined as the time starting from the moment of applying the composition to the surface to be disinfected after which the number of microbes per unit area is reduced by a factor of 10000 (i.e. a 4 log reduction),

wherein the initial number of microbes preferably exceeds $10^3$, more preferably $10^5$, and even more preferably $10^7$ microbes per square centimeter. Such tests may for instance be performed as described in WO 2010/046238, or as described in European Standards EN 13697:2001 and EN 1500:1997.

*Compositions according to the second aspect of the invention*

According to the second aspect of the invention, there is also provided an antimicrobial composition comprising:

a. 0.001 to 5% by weight of one or more substituted phenols,
b. a carrier, and
c. from 1 to 80% by weight of one or more surfactants;

and wherein the one or more substituted phenols have the following structure

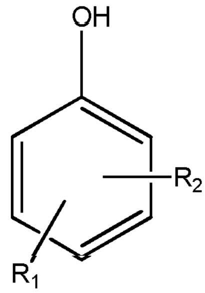

wherein $R_1$ is a cyclopentyl group or a cyclopentenyl group,
and wherein $R_2$ is independently selected from the group consisting of hydrogen, linear or branched $C_1$ to $C_5$ alkyl, and linear or branched $C_2$ to $C_5$ alkenyl.

The antimicrobial composition according to the second aspect of the invention preferably is suitable for use in the method according to the first aspect of the invention.

Therefore, similar to the antimicrobial composition to be applied in the method of the first aspect of the invention, the antimicrobial composition according to the second aspect of the invention comprises 0.001 to 5% by weight of the one or more substituted phenols. The composition comprises preferably 0.005 to 4.5 wt-%, more preferably 0.01 to 4 wt-%, even more preferably 0.02 to 3 wt-%, yet more preferably 0.03 to 2 wt-%, still more preferably 0.04 to 1 wt-%, even more preferably 0.05 to 0.75 wt-% and still more preferably 0.1 to 0.5 wt-% of the one or more substituted phenols.

The substituted phenol may be a single compound or may be a mixture of the selected substituted phenols. In case the composition according to the invention comprises a mixture of such substituted phenols, the mixture preferably comprises at least 30%, more preferably at least 50%, even more preferably at least 70% and still more preferably at least 90% by weight of one substituted phenol with respect to the total weight of the substituted phenols.

The substituent $R_1$ is a cyclopentyl group or a cyclopentenyl group. Preferably, $R_1$ is a cyclopentyl group.

The substituent $R_2$ is independently selected from the group consisting of hydrogen, linear or branched $C_1$ to $C_5$ alkyl, and linear or branched $C_2$ to $C_5$ alkenyl.
Preferably, $R_2$ is selected from the group consisting of hydrogen, linear or branched $C_1$ to $C_3$ alkyl, and linear or branched $C_2$ to $C_3$ alkenyl. More preferably, $R_2$ is hydrogen or methyl. Even more preferably, $R_2$ is hydrogen.

Combinations of preferred $R_1$ and preferred $R_2$ substituents are particularly preferred. Therefore, it is particularly preferred that the one or more substituted phenols are selected from the group consisting of 2-cyclopentylphenol, 4-cyclopentyl phenol, and mixtures thereof.

The structures (and examples thereof) of the substituted phenols of the composition according to the second aspect of the invention are as described above in relation to the substituted phenols of the composition to be applied in the method according to the first aspect of the invention.

The carrier in the composition according to the second aspect of the invention can be any carrier as described below.

*Additional features of the antimicrobial compositions*

The explanations, definitions and further features of antimicrobial compositions described below apply both to the compositions applicable in step (i) of the method according to the first aspect of the invention and to the antimicrobial

compositions according to the second aspect of the invention.

*Carrier*

The antimicrobial compositions according to step (i) of the first aspect of the invention and according to the second aspect of the invention comprise a carrier. The carrier is preferably selected from the group consisting of water, oil, solvent, inorganic particulate material, starch and mixtures thereof. The carrier is preferably from 0.1 to 99% by weight of the composition. The antimicrobial composition may be in form of a solid, liquid, gel, paste or soft solid and the carrier may be selected by a person skilled in the art depending on the format of the antimicrobial composition.

Examples of suitable inorganic particulate materials include clay, talc, calcite, dolomite, silica, and aluminosilicate. Examples of suitable oils include mineral oils, vegetable oils, and petroleum-derived oils and waxes. Examples of solvents include alcohols (in particular ethanol and isopropanol), ethers and acetone. The starch may be natural starch obtained from food grains or may be a modified starch.

Particularly preferred carriers are water or oil/solvent and even more preferred is a carrier that is a mixture of water and oil. Thus, in many of the envisaged applications like personal care/washing, oral care and hard surface cleaning, the antimicrobial composition may be formulated with either an aqueous base or a purely oil/solvent base. Compositions with an aqueous base (water being the carrier), may also for instance be products in gel format. Compositions with a purely oil/solvent base may for instance be products in anhydrous stick form or propellant-containing products.

Thus, the antimicrobial composition may for instance, preferably be an antimicrobial anhydrous stick personal care composition on a purely oil/solvent base wherein the composition has a water content of less than 0.01% by weight, and wherein the composition preferably is free of water. Alternatively, the antimicrobial composition may for instance, preferably be an antimicrobial propellant-drivable personal care composition, also comprising a propellant.

However, the most preferred product format has an emulsion base (water and oil being the carrier), e.g. soap products in liquid, solid, lotion or semisolid form for hand wash, face wash, body wash, or shaving applications; toothpaste/dentifrices for oral care applications or products for hard surface cleaning in bars or liquids form.

*Surfactants*

The antimicrobial composition according to the first aspect of the invention preferably comprises from 1 to 80% by weight of one or more surfactants. Surfactants may for instance advantageously contribute to the cleaning efficacy or the formulation stability of a composition.

Thus, the antimicrobial composition according to the second aspect of the invention comprises

a. 0.001 to 5% by weight of the one or more substituted phenols according to the invention,
b. a carrier, and
c. from 1 to 80% by weight of one or more surfactants.

In general, the surfactants may for instance be chosen from the surfactants described in well-known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch", H. Stache, 2nd Edn., Carl Hauser Verlag, 1981. Any type of surfactant, i.e. anionic, cationic, nonionic, zwitterionic or amphoteric can be used. Preferably, the surfactant is anionic, nonionic, or a mixture of anionic and nonionic surfactants. More preferably, the surfactant is anionic.

A particularly preferred surfactant is soap. Soap is a suitable surfactant for personal washing applications of the method of disinfecting a surface and the antimicrobial composition according to the invention. The soap is preferably $C_8$-$C_{24}$ soap, more preferably a $C_{10}$-$C_{20}$ soap and most preferably $C_{12}$-$C_{16}$ soap. The soap may or may not have one or more carbon-carbon double bonds or triple bonds. The cation of the soap can for instance be an alkali metal, alkaline earth metal or ammonium. Preferably, the cation of the soap is selected from sodium, potassium or ammonium. More preferably the cation of the soap is sodium or potassium.

The soap may be obtained by saponifying a fat and/or a fatty acid. The fats or oils may be fats or oils generally used in soap manufacture, such as tallow, tallow stearines, palm oil, palm stearines, soya bean oil, fish oil, castor oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soyabean, castor etc. The fatty acid soaps can also be synthetically prepared (e.g. by the oxidation of petroleum or by the hydrogenation of carbon monoxide by the Fischer-Tropsch process). Resin acids, such as those present in tall oil, may be used. Naphthenic acids are also suitable.

Tallow fatty acids can be derived from various animal sources and generally comprise about 1 to 8% myristic

acid, about 21 to 32 wt-% palmitic acid, about 14 to 31 wt-% stearic acid, about 0 to 4 wt-% palmitoleic acid, about 36 to 50 wt-% oleic acid and about 0 to 5 wt-% linoleic acid. A typical distribution is 2.5 wt-% myristic acid, 29 wt-% palmitic acid, 23 wt-% stearic acid, 2 wt-% palmitoleic acid, 41.5 wt-% oleic acid, and 3 wt-% linoleic acid. Other similar mixtures, such as those from palm oil and those derived from various animal tallow and lard are also included.

Coconut oil refers to fatty acid mixtures having an approximate carbon chain length distribution of 8 wt-% $C_8$, 7 wt-% $C_{10}$, 48 wt-% $C_{12}$, 17 wt-% $C_{14}$, 8 wt-% $C_{16}$, 2 wt-% $C_{18}$, 7 wt-% oleic and 2 wt-% linoleic acids (the first six fatty acids listed being saturated). Other sources having similar carbon chain length distributions, such as palm kernel oil and babassu kernel oil, are included within the term coconut oil.

A typical fatty acid blend consists of 5 to 30 wt-% coconut fatty acids and 70 to 95 wt-% fatty acids ex hardened rice bran oil. Fatty acids derived from other suitable oils/fats such as groundnut, soybean, tallow, palm, palm kernel, etc. may also be used in other desired proportions. The soap, when present in solid forms of the present invention, is present in an amount of 30 to 80%, preferably from 50 to 80%, more preferably 55 to 75% by weight of the composition. The soap, when present in liquid forms of the composition is present in 0.5 to 20%, preferably from 1 to 10% by weight of the composition.

The method of disinfecting a surface according to the first aspect of the invention and the antimicrobial composition according to the second aspect of the invention are also useful in hard surface cleaning applications. In such applications, preferred surfactants are nonionic surfactants, such as $C_8$-$C_{22}$, preferably $C_8$-$C_{16}$ fatty alcohol ethoxylates, comprising between 1 and 8 ethylene oxide groups when the product is in the liquid form. When the product for hard surface cleaning applications is in the solid form, surfactants are preferably selected from primary alkyl sulphates, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucosides may also be present in the composition, preferably those having a carbon chain length between C6 and C16. Suitable surfactant concentrations in liquid forms of hard surface cleaning application are generally from about from 0.5 to 10%, preferably from 1 to 5 % by weight of the composition. In solid compositions, surfactant is preferably present in 5 to 40%, preferably from 10 to 30% by weight of the composition.

The method of disinfecting a surface according to the first aspect of the invention and the antimicrobial composition according to the second aspect of the invention are also useful in oral care compositions e.g. in a dentifrice/ toothpaste or an oral rinse product. In such applications, preferred surfactants are anionic, nonionic or amphoteric in nature, preferably anionic or amphoteric. The anionic surfactant is preferably an alkali metal alkyl sulphate, more preferably a sodium lauryl sulphate (SLS). Mixtures of anionic surfactants may also be employed. The amphoteric surfactant is preferably a betaine, more preferably an alkylamidopropyl betaine (wherein the alkyl group is a linear $C_{10}$-$C_{18}$ chain), and most preferably is cocoamidopropyl betaine (CAPB). Mixtures of amphoteric surfactants may also be employed. Suitable surfactant concentrations in oral care application are generally from about 2% to about 15%, preferably from about 2.2% to about 10%, more preferably from about 2.5 to about 5% by weight of the total composition.

Thus, it is highly preferred that the antimicrobial compositions include soap, alkyl sulphate or linear alkyl benzene sulphonate as the surfactants. More preferably, the surfactant is a soap, an alkyl sulphate or a linear alkyl benzene sulphonate.

The antimicrobial composition may be in form of a solid, a liquid, a gel or a paste. A person skilled in the art can prepare compositions in various formats by choosing one or more carrier materials and/or surfactant. The antimicrobial compositions of the present invention are useful for cleansing and care, in particular for skin cleansing and skin care. It is envisaged that the antimicrobial composition can be used as a leave-on product or a wash-off product, preferably a wash-off product. The antimicrobial composition of the present invention can also be used for cleansing and care of hard surfaces such as glass, metal, plastic and the like.

*Liquid and solid compositions*

A particularly preferred carrier in the antimicrobial composition according to the first or the second aspect of the invention is water. When water is present, it is preferably present in at least 1%, more preferably at least 2%, further more preferably at least 5% by weight of the composition. When water is the carrier, a preferred liquid antimicrobial composition according to the first or the second aspect of the invention comprises:

a. 0.05 to 5% by weight of the one or more substituted phenols,
b. 10 to 99.9% by weight of water, and;
c. 1 to 30% by weight of surfactant.

The liquid antimicrobial composition is useful for skin cleansing, in particular for hand wash or face wash.

When water is the carrier, a preferred solid antimicrobial composition according to the invention comprises:

a. 0.05 to 5% by weight of the one or more substituted phenols,
b. 5 to 30% by weight of water, and;
c. 30 to 80% by weight of surfactant.

The solid antimicrobial composition is preferably in form of a shaped solid, more preferably a bar. The solid antimicrobial composition is particularly useful for skin cleansing in particular for hand wash or a face wash.

Such a bar-shaped solid antimicrobial composition may for instance be a soap bar. Soap bar compositions are well-known and may be similar to the following non-limiting example composition, comprising 75.6 wt-% of anhydrous sodium soap, 1.0 wt-% of glycerine, 0.5 wt-% of sodium carbonate, 0.2 wt-% of EHDP (ethane-1-hydroxy-1,1-disphosphonate) acid, 0.04 wt-% of EDTA (ethylenediaminetetraacetic acid) tetrasodium salt, 8.5 wt-% of hydrated magnesium silicate (Talc), 0.7 wt-% of sodium chloride, 0.05 wt-% of dyes, 0.75 wt-% perfume, 0.05 to 10 wt-% of preservatives and antimicrobial agents including the substituted phenols according to the present invention, and water up to 100 wt-%.

Alternatively, inorganic particulate material is also a suitable carrier. When inorganic particulate material is the carrier, the antimicrobial composition is in a solid form. Preferably the inorganic particulate material is talc. When the inorganic particulate material is talc, the solid antimicrobial composition is particularly useful as a talcum powder for application on face or body.

According to another alternative, a solvent is a preferred carrier. Although any solvent can be used, alcohol is a preferred solvent. Short chain alcohols -in particular ethanol, propanol and isopropanol- are particularly preferred as carrier for an antimicrobial wipe or an antimicrobial hand sanitiser composition.

Solvents like ethanol and isopropanol generally show antimicrobial efficacy themselves. However, they are also volatile and may readily evaporate during application of the composition. Thus, their levels on the surface that is treated might even reduce until below the minimum level required for antimicrobial action, before the minimum period needed for disinfection has passed. In contrast, the substituted phenols according to the present invention are much less volatile and may therefore yield prolonged antimicrobial action after applying them to the skin.

*Additional ingredients*

The composition according to the first or second aspect of the invention may further comprise various additional ingredients known to a person skilled in the art. Such additional ingredients include but are not limited to: perfumes, pigments, preservative, emollients, sunscreens, emulsifiers, gelling agents, or thickening agents, humectants (e.g. glycerine, sorbitol), sequestrants (e.g. EDTA) or polymers (e.g. cellulose derivatives for structuring such as methyl cellulose).

Some of the substituted phenols according to the invention may contribute to the olfactory properties of the composition. Although some of these compounds might be applied for instance in perfume compositions, the present invention is directed towards antimicrobial compositions. Therefore, the composition is preferably not a perfume composition. Here, a perfume composition is defined as a composition comprising a plurality of olfactory components, where these components are solely intended to provide the composition with a harmonious scent.

**EXAMPLES**

The invention is illustrated by the following non-limiting examples.

**Example 1: Assessment of antimicrobial efficacy**

The efficacies of antimicrobial agents can be usefully compared by determining the Minimum Biocidal Concentration (MBC). The MBC is defined as the absolute lowest concentration of actives that provides complete kill (zero bacterial growth) in a particular test environment.

*Experimental method*

Antimicrobial efficacy is tested against a representative pathogenic bacterial organism, Gram-negative *Escherichia coli.* Concentrations of actives are expressed in terms of the percentage weight/volume (%w/v) throughout Example 1.

*Bacterial stock*

An overnight culture of *Escherichia coli* (10536 strain) was prepared in 50 ml total volume of TSB broth, grown for ca. 18 hrs at 37°C and shaken at 150 rpm. 1 ml of this overnight *E. coli* culture was transferred to 50 ml of fresh TSB broth and incubated at 37°C at 150 rpm for ca. 4 hours. This culture was separated into equal volumes and centrifuged

at 4000 rpm for 15 minutes, washed with sterile saline (0.85% NaCl), centrifuged once more and re-suspended in saline to give a final concentration of 0.8 $OD_{620}$ equivalent to about $10^8$ cells per millilitre for this particular organism. Here, $OD_{620}$ indicates the absorbance of a sample in a cuvette of 1.0 cm path length at a wavelength of 620 nm. This bacterial stock was used for assaying against antimicrobial actives (in triplicate).

*Protocol*

The following assay describes the testing of 8 materials using 6 dilutions across half of a 96-well micro titre plate (MTP). Using this approach it is possible to assay 16 actives (without replicates) with one full dilution plate, replicating this set up in two halves of the plate columns, 1-6 and 7-12.

1 M solutions of the test actives were prepared in dimethylsulphoxide (DMSO). Stock solutions of the actives at 1.11 times the desired final concentration were prepared by diluting the DMSO solutions in water, so that for example a 0.89% w/v solution was prepared for a desired "in test" concentration of 0.8% w/v in order to allow for the further dilution of the active when the bacterial suspension is added (dilution from 270μl to 300 μl), as described below.

Aliquots (270 μl) of the materials at 1.11 times the final concentration were dispensed into the wells of the MTP along one column (A1-H1). This MTP was labelled as the "Screening plate".

In another MTP, labelled as the "Dilution plate", 270μl of D/E neutralising solution from DIFCO Composition was added to column 1. The composition of the neutralising solution was as follows: pancreatic digest of casein, 5.0g/L; Yeast Extract, 2.5 g/L; Dextrose, 10 g/L, sodium thioglycollate, 1.0 g/L, sodium thiosulphate, 6.0 g/L; sodium bisulphite, 2.5 g/L; Polysorbate 80, 5.0 g/L; lecithin 7.0 g/L; bromocresol purple, 0.02 g/L with a pH in the range 7.6±0.2.

270μl of tryptone diluent solution was added to all the remaining wells of the Dilution MTP (columns 2-6).

Bacterial stock (30 μl) was then added to the prepared 270 μl of the solution of actives in the Screening Plate and mixed, using a multichannel pipette with 8 tips to aspirate and dispense the same volume of bacterial stock in parallel to 8 wells in rows A-H. After a contact time of 15 seconds, the mixtures were quenched by transferring 30μl volumes of the mixtures into the 270μl D/E neutralising solution in the prepared dilution plate, using aspiration to mix. After exactly 5 minutes in the D/E neutralising solution, 30μl volumes were transferred from column 1 to column 2 of the Dilution MTP and mixed, before transferring further 30μl volumes from column 2 into column 3. This process was repeated serially diluting the bacteria across the plate to column 6.

30μl volumes from each well in the Dilution MTP were transferred onto pre-labelled segment of Tryptone Soya Agar (TSA) plates starting from the lowest bacterial concentration (highest dilution, column 6) to the highest bacterial concentration (column 1). The TSA plates were allowed to stand for ca. 2 hours so that the 30μl inocula spots could dry and the plates were then inverted and incubated overnight at 37°C before enumerating the bacterial colonies at the labelled dilutions to determine the effects of the actives on bacterial growth.

*Calculation of results*

Mean bacterial survival numbers $N_{MBS}$ (expressed in Log CFU/ml) are obtained by first determining the segment of the TSA plate where the number of bacterial colonies is countable. From the colony number in this segment, $N_{MBS}$ is calculated by the formula:

$$N_{MBS} = \log\{ N_{col} \cdot 10^{DF} \cdot 100 / 3 \}$$

Here, $N_{col}$ is the colony count, and DF is the dilution factor taken from the MTP-well corresponding to the TSA plate segment (i.e. DF may range from 1 for the quench, to 6 for the highest dilution). The factor 100/3 is a conversion factor from the volume of the inocula spot to one millilitre.

Every assay test was performed in triplicate. The reported mean bacterial survival results are the average of such a triplet, the error is the corresponding standard deviation.

Thus, a value of $N_{MBS}$ of about 7 corresponds to a count of about 3 colonies from the fifth dilution well, i.e. with DF = 5. Such a count of about 7 is generally observed when bacteria are exposed to non-biocidal materials. In case no surviving colonies are observed in any segment of the TSA plate, this is interpreted as complete kill and a value of $N_{MBS}$=0 is reported.

*Validation*

All test results were validated by running every test assay in parallel with four control experiments on the same MTP. All control experiments are executed exactly according to the above protocol, but with the following active ingredients:

A 0.025 %(w/v) thymol
B 0.15 %(w/v) alpha-terpineol
C 0.025 %(w/v) thymol + 0.15 %(w/v) alpha-terpineol
D no active component

The control experiments A, B and D validate a test assay by not showing bacterial kill, whereas control experiment C, comprising a combination of thymol and alpha-terpineol according to WO 2010/046238 A1 validates a test assay by showing complete bacterial kill.

A reference experiment according to the above protocol, but without active component, showed that DMSO does not affect bacterial growth at the concentrations present in the test solutions in this protocol (<5 % (w/v)), as can be seen in Table 3.

**Table 3**

| DMSO in water (% w/v) | Mean bacterial survival [log CFU/ml] | Standard deviation |
|---|---|---|
| 4.5 | 8.2 | 0.1 |
| 3.6 | 8.4 | 0.2 |
| 2.7 | 8.2 | 0.1 |
| 1.8 | 8.5 | 0.2 |
| 0.9 | 8.6 | 0.1 |
| 0.0 | 8.5 | 0.1 |

*Results*

The above method was applied to assess the antibacterial efficacy of the substituted phenols according to the present invention. For each of the below examples, Table 4 shows the antibacterial activities of the substituted phenols.

**Table 4:** Antibacterial activities of substituted phenols

| Example | **Concentration of** substituted phenol $C_{phenol}$ (% w/v) | $N_{MBS}$ [log CFU/ml] | Standard deviation |
|---|---|---|---|
| 1:1 | 0.15% 2-cyclopentylphenol | 0 | 0 |
| 1:2 | 0.08% 4-cyclopentylphenol | 0 | 0 |
| 1:3* | 0.5% 2-cyclohexyl-5-methylphenol | 0 | 0 |
| 1:4* | 0.3% 2-cyclohexyl-5-methylphenol | 5.55 | 0.082 |
| * Examples (1:3) and (1:4) are comparative examples | | | |

The compound 2-cyclohexyl-5-methylphenol is a compound according to the prior art.

*Antimicrobial compositions*

As described above, the MBC for an active can be defined as the lowest measured concentration of the active that provides zero bacterial bacterial survival for the particular bacteria in the particular medium. For 2-cyclohexyl-5-methylphenol, the comparative compositions (1:3) and (1:4), show that the MBC is between 0.5% (w/v) and 0.3 % (w/v). The same analysis has been carried out for the substituted phenols according to the present invention and is summarised in Table 5 below. The reported MBC values for the substituted phenols constitute upper boundaries to the MBC in the particular medium used in these examples.

**Table 5:** Minimum Biocidal concentrations of antimicrobial components

| Component | MBC (% w/v) |
|---|---|
| 2-cyclopentylphenol | 0.15 |

(continued)

| Component | MBC (% w/v) |
|---|---|
| 4-cyclopentylphenol | 0.08 |
| 2-cyclohexyl-5-methylphenol | 0.5 |

It is clear from the data in Table 5 that the substituted phenols according to the present invention are more efficacious antimicrobials than 2-cyclohexyl-5-methylphenol and can be used at lower concentrations.

## Claims

**1.** A non-therapeutic method of disinfecting a surface comprising the steps of

a. applying to the surface an antimicrobial composition comprising

i. 0.001 to 5% by weight of one or more substituted phenols, and
ii. a carrier; and

b. removing the composition from the surface;

wherein the one or more substituted phenols have the following structure

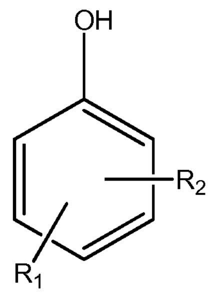

wherein

$R_1$ is a cyclopentyl group or a cyclopentenyl group,
and wherein
$R_2$ is independently selected from the group consisting of

hydrogen,
linear or branched $C_1$ to $C_5$ alkyl, and
linear or branched $C_2$ to $C_5$ alkenyl.

**2.** A method according to claim 1, wherein $R_2$ is selected from the group consisting of

hydrogen,
linear or branched $C_1$ to $C_3$ alkyl, and
linear or branched $C_2$ to $C_3$ alkenyl.

**3.** A method according to claim 2, wherein $R_2$ is hydrogen or methyl.

**4.** A method according to any one of claims 1 to 3, wherein $R_1$ is a cyclopentyl group.

**5.** A method according to claim 4, wherein the one or more substituted phenols are selected from the group consisting of 2-cyclopentylphenol, 4-cyclopentylphenol, and mixtures thereof.

6. A method according to any one of claims 1 to 5, wherein the antimicrobial composition comprises from 1 to 80% by weight of one or more surfactants.

7. A method according to any one of claims 1 to 6, wherein the surface is skin.

8. A method according to any one of claims 1 to 7, wherein the disinfection time T of said method is less than 300 seconds, wherein T is defined as the time that elapses from the moment of adding the antimicrobial composition to a microbial culture until the number of microbes per unit volume of the culture is reduced by a factor of 100 000.

9. An antimicrobial composition comprising:

a. 0.001 to 5% by weight of one or more substituted phenols,
b. a carrier, and
c. from 1 to 80% by weight of one or more surfactants;

and wherein the one or more substituted phenols have the following structure

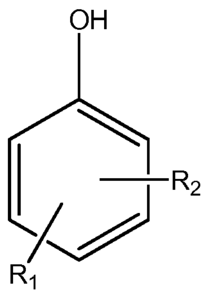

wherein

$R_1$ is a cyclopentyl group or a cyclopentenyl group,
and wherein
$R_2$ is independently selected from the group consisting of

hydrogen,
linear or branched $C_1$ to $C_5$ alkyl, and
linear or branched $C_2$ to $C_5$ alkenyl.

10. An antimicrobial composition according to claim 9, wherein $R_2$ is selected from the group consisting of

hydrogen,
linear or branched $C_1$ to $C_3$ alkyl, and
linear or branched $C_2$ to $C_3$ alkenyl.

11. An antimicrobial composition according to claim 10 wherein $R_2$ is hydrogen or methyl.

12. An antimicrobial composition according to claim 11, wherein the one or more substituted phenols are selected from the group consisting of 2-cyclopentylphenol, 4-cyclopentyl phenol, and mixtures thereof.

13. An antimicrobial composition according to any one of claims 9 to 12 comprising from 1 to 99% by weight of the carrier, wherein said carrier is selected from the group consisting of water, ethanol and isopropanol and mixtures thereof.

**Patentansprüche**

1. Nicht-therapeutisches Verfahren zum Desinfizieren einer Oberfläche, das die Schritte umfasst:

a. Aufbringen einer antimikrobiellen Zusammensetzung, die

i. 0,001 bis 5 Gewichts-% eines oder mehrerer substituierter Phenole und
ii. einen Träger

umfasst, auf die Oberfläche und
b. Entfernen der Zusammensetzung von der Oberfläche,

wobei das eine oder die mehreren substituierten Phenole die folgende Struktur

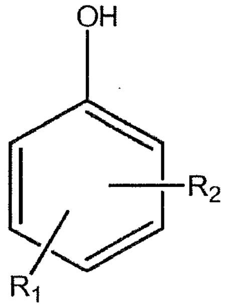

aufweisen,
worin

$R_1$ eine Cyclopentyl- oder eine Cyclopentenyl-Gruppe darstellt und worin
$R_2$, unabhängig, unter der aus Wasserstoff, linearem oder verzweigtem $C_1$- bis $C_5$-Alkyl und linearem oder verzweigtem $C_2$- bis $C_5$-Alkenyl bestehenden Gruppe ausgewählt ist.

**2.** Verfahren nach Anspruch 1, wobei $R_2$ aus der aus Wasserstoff, linearem oder verzweigtem $C_1$- bis $C_3$-Alkyl und linearem oder verzweigtem $C_2$- bis $C_3$-Alkenyl bestehenden Gruppe ausgewählt ist.

**3.** Verfahren nach Anspruch 2, wobei $R_2$ Wasserstoff oder Methyl ist.

**4.** Verfahren nach irgendeinem der Ansprüche 1 bis 3, wobei $R_1$ eine Cyclopentylgruppe darstellt.

**5.** Verfahren nach Anspruch 4, wobei das eine oder die mehreren substituierten Phenole aus der aus 2-Cyclopentylphenol, 4-Cyclopentylphenol und Mischungen davon bestehenden Gruppe ausgewählt ist.

**6.** Verfahren nach irgendeinem der Ansprüche 1 bis 5, wobei die antimikrobielle Zusammensetzung 1 bis 80 Gewichts-% eines oder mehrerer Tenside umfasst.

**7.** Verfahren nach irgendeinem der Ansprüche 1 bis 6, wobei die Oberfläche Haut darstellt.

**8.** Verfahren nach irgendeinem der Ansprüche 1 bis 7, wobei die Desinfektionszeit T des Verfahrens weniger als 300 Sekunden beträgt, wobei T als die Zeit definiert wird, die von dem Moment der Zugabe der antimikrobiellen Zusammensetzung zu einer mikrobiellen Kultur verstreicht, bis die Anzahl der Mikroben pro Einheitsvolumen der Kultur um einen Faktor 100 000 reduziert ist.

**9.** Antimikrobielle Zusammensetzung, die

a. 0,001 bis 5 Gewichts-% eines oder mehrerer substituierter Phenole,
b. einen Träger und
c. 1 bis 80 Gewichts-% eines oder mehrerer Tenside

umfasst,
und worin das eine oder die mehreren substituierten Phenole die folgende Struktur

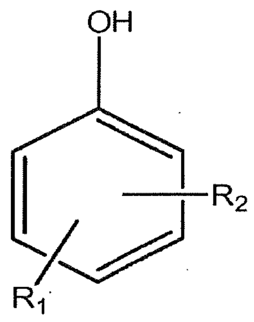

aufweisen,
wobei

$R_1$ eine Cyclopentyl- oder eine Cyclopentenyl-Gruppe darstellt und worin
$R_2$, unabhängig, aus der aus Wasserstoff, linearem oder verzweigtem $C_1$- bis $C_5$-Alkyl und linearem oder verzweigtem $C_2$- bis $C_5$-Alkenyl bestehenden Gruppe ausgewählt ist.

**10.** Antimikrobielle Zusammensetzung nach Anspruch 9, wobei $R_2$ aus der aus Wasserstoff, linearem oder verzweigtem $C_1$- bis $C_3$-Alkyl und linearem oder verzweigtem $C_2$- bis $C_3$-Alkenyl bestehenden Gruppe ausgewählt ist.

**11.** Antimikrobielle Zusammensetzung nach Anspruch 10, wobei $R_2$ Wasserstoff oder Methyl ist.

**12.** Antimikrobielle Zusammensetzung nach Anspruch 11, wobei das eine oder die mehreren substituierten Phenole aus der aus 2-Cyclopentylphenol, 4-Cyclopentylphenol und Mischungen davon bestehenden Gruppe ausgewählt sind.

**13.** Antimikrobielle Zusammensetzung nach irgendeinem der Ansprüche 9 bis 12, die 1 bis 99 Gewichts-% des Trägers umfasst, wobei der Träger aus der aus Wasser, Ethanol und Isopropanol und Mischungen davon bestehenden Gruppe ausgewählt ist.

## Revendications

**1.** Procédé non-thérapeutique de désinfection d'une surface comprenant les étapes de

a. application à la surface d'une composition antimicrobienne comprenant

i. de 0,001 à 5 % en masse d'un ou plusieurs phénols substitués, et
ii. un support ; et

b. élimination de la composition de la surface ;

dans lequel les un ou plusieurs phénols substitués ont la structure suivante

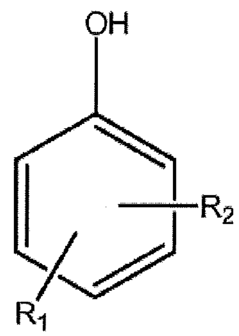

où

$R_1$ est un groupe cyclopentyle ou un groupe cyclopenténlyle, et où
$R_2$ est indépendamment choisi dans le groupe constitué de

l'hydrogène,
un groupe alkyle en $C_1$ à $C_5$ linéaire ou ramifié, et
un groupe alcényle en $C_2$ à $C_5$ linéaire ou ramifié.

2. Procédé selon la revendication 1, dans lequel $R_2$ est choisi dans le groupe constitué de

l'hydrogène,
un groupe alkyle en $C_1$ à $C_3$ linéaire ou ramifié, et
un groupe alcényle en $C_2$ à $C_3$ linéaire ou ramifié.

3. Procédé selon la revendication 2, dans lequel $R_2$ est l'hydrogène ou le groupe méthyle.

4. Procédé selon l'une quelconque des revendications 1 à 3, dans lequel $R_1$ est un groupe cyclopentyle.

5. Procédé selon la revendication 4, dans lequel les un ou plusieurs phénols substitués sont choisis dans le groupe constitué de 2-cyclopentylphénol, 4-cyclopentylphénol, et mélanges de ceux-ci.

6. Procédé selon l'une quelconque des revendications 1 à 5, dans lequel la composition antimicrobienne comprend de 1 à 80 % en masse d'un ou plusieurs tensioactifs.

7. Procédé selon l'une quelconque des revendications 1 à 6, dans lequel la surface est la peau.

8. Procédé selon l'une quelconque des revendications 1 à 7, dans lequel la durée de désinfection T dudit procédé est inférieure à 300 secondes, où T est définie comme le temps qui s'écoule à partir du moment de l'addition de la composition antimicrobienne à une culture microbienne jusqu'à ce que le nombre de microbes par volume unitaire de la culture est réduit d'un facteur 100 000.

9. Composition antimicrobienne comprenant :

a. de 0,001 à 5 % en masse d'un ou plusieurs phénols substitués,
b. un support, et
c. de 1 à 80 % en masse d'un ou plusieurs tensioactifs ;

et dans laquelle les un ou plusieurs phénols substitués présentent la structure suivante

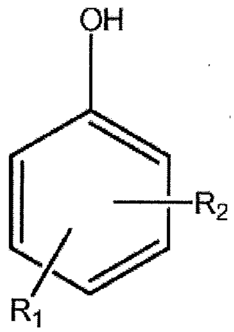

où

$R_1$ est un groupe cyclopentyle ou un groupe cyclopenténlyle, et où
$R_2$ est indépendamment choisi dans le groupe constitué de

l'hydrogène,

un groupe alkyle en $C_1$ à $C_5$ linéaire ou ramifié, et
un groupe alcényle en $C_2$ à $C_5$ linéaire ou ramifié.

**10.** Composition antimicrobienne selon la revendication 9, dans laquelle $R_2$ est choisi dans le groupe constitué de

l'hydrogène,
un groupe alkyle en $C_1$ à $C_3$ linéaire ou ramifié, et
un groupe alcényle en $C_2$ à $C_3$ linéaire ou ramifié.

**11.** Composition antimicrobienne selon la revendication 10, dans laquelle R2 est l'hydrogène ou un groupe méthyle.

**12.** Composition antimicrobienne selon la revendication 11, dans laquelle les un ou plusieurs phénols substitués sont choisis dans le groupe constitué de 2-cyclopentylphénol, 4-cyclopentylphénol, et mélanges de ceux-ci.

**13.** Composition antimicrobienne selon l'une quelconque des revendications 9 à 12 comprenant de 1 à 99 % en masse du support, dans laquelle ledit support est choisi dans le groupe constitué d'eau, éthanol et isopropanol et mélanges de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 1137 M
- FR 1313717
- BE 591064
- DE 2263126 A1
- WO 2010046238 A
- WO 2010046238 A1

### Non-patent literature cited in the description


- **P A GODDARD ; K A MCCUE.** Disinfection, Sterilisation and Preservation. Lippincott, Williams and Wilkins, 2001, 255-282
- **JP VOETS et al.** *J. Appl. Bact.,* 1976, vol. 40, 67-72
- **J JENSEN.** Chlorophenols in the terrestrial environment. *Reviews of environmental contamination and toxicology,* 1996, vol. 146, 25-51
- **P. PHUKAN ; A. SUDALAI.** J. Chem. Soc. Perkin Trans, 1999, 3015
- **S. H. DAI ; C. Y. LIN ; D. V. RAO ; F. A. STUBER ; P. S. CARLETON ; H. ULRICH.** *J. Org. Chem.,* 1985, vol. 50, 1722
- **SCHWARTZ ; PERRY.** Surface Active Agents. Interscience, 1949, vol. 1
- **SCHWARTZ, PERRY ; BERCH.** SURFACE ACTIVE AGENTS. Interscience, 1958, vol. 2
- McCutcheon's Emulsifiers and Detergents. Manufacturing Confectioners Company
- **H. STACHE.** Tenside-Taschenbuch. Carl Hauser Verlag, 1981